# EUROPEAN PATENT APPLICATION

(11) **EP 4 517 336 A1**
(43) Date of publication of application: **05.03.2025**
(21) Application number: 23724897.6
(22) Date of filing: 28.04.2023
(51) Int. Cl.: G01Q 30/04, G01Q 60/32, G01Q 60/42, G06V 20/69, G16B 15/00, G06N 3/08, G06N 3/094, G06F 18/24

(54) **COMPUTER-IMPLEMENTED METHOD FOR IDENTIFYING AN ORGANIC MOLECULE FROM ATOMIC FORCE MICROSCOPY IMAGES BY GENERATING A TWO-DIMENSIONAL (2D) STRUCTURAL REPRESENTATION OF SAID MOLECULE, COLORED WITH THE RGB (RED, GREEN, BLUE) COLOR SYSTEM, IN THE FORM OF A BALL-AND-STICK IMAGE**

(30) Priority: 29.04.2022 ES 202230396
(71) Applicant: Universidad Autónoma de Madrid, 28049 Madrid (ES)
(72) Inventor: PÉREZ PÉREZ, Rubén, 28049 Madrid (ES); CARRACEDO COSME, Jaime, 28049 Madrid (ES)
(74) Representative: Pons
(86) International application number: PCT/ES2023/070275
(87) International publication number: WO 2023/209263

(57) **Abstract**

The invention relates to a computer implemented method for identifying an organic molecule from Atomic Force Microscopy (AFM) images and generating a 2D colored RGB structural representation of said organic molecule, particularly a two-dimensional (2D) colored RGB structural representation in the form of a ball-and-stick depiction of the atoms and the distance between said atoms using a trained Conditional Generative Adversarial Network (CGAN). The present invention is therefore of interest in the areas of nanotechnology, particularly in areas related to on-surface chemical reactions, and therefore of interest for the AFM users and manufacturers.

## Description

The invention relates to a computer implemented method for identifying an organic molecule from Atomic Force Microscopy (AFM) images by generating a 2D colored RGB structural representation of said organic molecule in the form of a ball-and-stick depiction by means of a trained Conditional Generative Adversarial Network (CGAN).

The present invention is therefore of interest in the areas of nanotechnology, particularly in areas related to on-surface chemical reactions, and therefore of interest for the AFM users and manufacturers.

### STATE OF ART

Molecules play an increasingly relevant role in modern societies. Besides their traditional use as key materials for the food, textile, chemical and pharmaceutical industries, the latest trend in nanotechnology see them as the ultimate miniaturization elements. Single molecules now act as electrical and optical devices or force actuators, driving the development of entire new research fields such as molecular electronics, energy harvesting and molecular motors. Besides the reduction in size and energy consumption, molecules offer the versatility of molecular structure and composition to fine tune the system to the intended function. Versatility requires novel synthesis routes, and radically new ideas like on-surface chemistry have been added to the more traditional strategies in solution chemistry. To date, the identification of molecular structure and composition relies heavily on spectroscopic methods like nuclear magnetic resonance (NMR), mass spectrometry (MS), X-ray diffraction that measure molecular ensembles. They are clearly not suitable for the characterization of the products of on-surface reactions, but they are also limited in the case of traditional solution-based synthesis.

Atomic Force Microscopy (AFM) in combination with dynamic operation modes has become one of the key tools for imaging and manipulation of materials and biological systems at the nanoscale. Operated in the frequency-modulation mode (FM), commonly known as Non-contact AFM, AFM achieves true atomic-scale resolution. The use of metal tips functionalized with a CO molecule at the tip apex, has provided access to the internal structure of molecules with totally unprecedented resolution. The main contrast mechanism for AFM with inert tips like CO is Pauli repulsion, that is due to the overlap of the electron densities of tip and sample. This repulsive force produces positive frequency shifts -changes in the oscillation frequency of the cantilever holding the tip due to the tip-sample interaction- that are observed as bright features in the constant height AFM images above atom positions and sizes and bonds (distance between atoms), reflecting the molecular structure. Increasingly accurate AFM simulation models have been developed to explain the observed image contrast. They have contributed to elucidate the role of the CO tilting, the influence of other contributions to the tip-sample interaction, like the electrostatic force, the role of the CO-metal tip charge distribution, and the interplay of the short-range chemical interaction and electrostatics in bond order discrimination and the imaging of intermolecular bonds.

High-resolution experimental (HR) AFM images, together with the ability to address individual molecules, have paved the way for the identification of natural products -like breitfussin A, where the structure of some of the fragments was known but methods like NMR failed to provide the global structure (K. Ø. Hanssen, et al., Angew. Chem. Int. Ed. 51, 12238 (2012)). HR-AFM is also key in the imaging of the intermediates (including radicals) and final products generated in on-surface reactions, shedding light into the formation processes and reaction pathways (S. Kawai, et al., ACS nano 11, 8122 (2017)). The technique has been able to resolve more than a hundred different types of molecules in asphaltenes, the solid component of crude oil (B. Schuler, G. Meyer, D. Peña, O. C. Mullins, L. Gross, J. Am. Chem. Soc. 137, 9870 (2015)). Molecular identification in all of the previous cases was supported by significant information about the nature of the molecules involved, as in the case of asphaltenes, where we were dealing essentially with polycyclic aromatic hydrocarbons based on C and H atoms. In spite of the wealth of information provided by HR-AFM experiments and these advances in the interpretation of the observed contrast, the complete identification of molecular systems, i.e. the determination of the structure and composition, solely based on HR-AFM images, without any prior information, remains an open problem.

Few works have tried to tackle this problem using Artificial Intelligence (AI) techniques (B. Alldritt, et al., Sci. Adv. 6, eaay6913 (2020) and J. Carracedo-Cosme, C. Romero-Muñiiz, R. Pérez, Nanomaterials 11, 1658 (2021)) to process AFM images. Deep Learning (DL) is nowadays routinely used to classify, interpret, describe and analyze images providing machines with capabilities that surpass human beings. DL ability to recognize patterns could in principle be exploited to characterize the structure of molecular systems. Gordon et al. (O. M. Gordon, J. E. Hodgkinson, S. M. Farley, E. L. Hunsicker, P. J. Moriarty, Nano Lett.20, 7688 (2020)) implemented a model to automate the detection of spatially correlated patterns in varied sets of AFM images of selforganised nanoparticles. However, the complete atom-by-atom identification poses a significant challenge, as the effects of both geometry and chemical composition contribute, in a very intricate way, to the determination of the 3D molecular charge density, that is ultimately responsible for the AFM contrast.

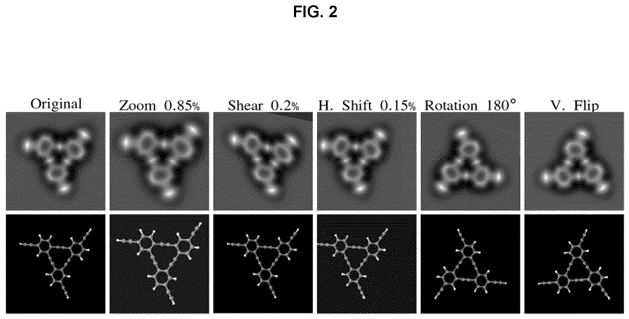

This complexity makes the task of molecular identification to go beyond the capability of the best human expert, as conclusively illustrated by the examples shown in Figure 2. The structure of the molecule in terms of rings and side groups is completely hidden by the chemical composition and its non-planar configuration. Alldritt et al. (B. Alldritt, et al., Sci. Adv. 6, eaay6913 (2020).) developed a Convolutional Neural Network (CNN) whose aim was to determine the molecular geometry from AFM images. The performance was excellent for the structure of quasi-planar molecules, even using the algorithm directly with experimental results. For 3D structures, they were able to recover information for the positions and sizes of the atoms closer to the tip, in a height range of 150 pm. However, the discrimination of functional groups produced non conclusive results.

In our previous work (J. Carracedo-Cosme, C. Romero-Muñiz, R. Pérez, Nanomaterials 11, 1658 (2021)), we showed the feasibility of performing a very accurate automatic molecular classification with DL techniques for a set of 60 planar molecules, that include the most common atomic species in organic chemistry, using their theoretically simulated AFM images. Furthermore, we proposed a Variational Autoencoder (VAE) based method to include the characteristic features of the experimental AFM images in the dataset, significantly increasing the accuracy of the model tested with experimental images. However, although this approach shows the potential to recognise both the structure and composition of molecules through AFM images, it does not come close to solving the global classification problem, since (i) classification in the usual sense with CNN just allows a finite-length output, i.e., only a finite number of structures can be classified, and (ii) we need to consider molecules with a non-planar adsorption configuration.

Therefore, it is needed to develop new methods for the identification of molecules processing AFM images.

### DESCRIPTION OF THE INVENTION

The object of the present invention is providing a computer implemented method for identifying an organic molecule from Atomic Force Microscopy (AFM) images by generating a two-dimensional (2D) colored RGB structural representation of said organic molecule using a trained Conditional Generative Adversarial Network (CGAN). The computer implemented method of the present invention generates a 2D RGB structural representation of the organic molecule in the form of a ball-and-stick depiction, wherein balls of different colors and sizes represent the different chemical atoms and sticks represent the bonds between the atoms and wherein each ball of the representation is centered on the position occupied by the atom it represents in the AFM images. The 2D RGB structural representation of the organic molecule in the form of a ball-and-stick depiction provides complete information on its structure and its chemical composition. In the present invention the different chemical nature of the atoms is represented by different colors and sizes proportional to the van der Waals radius of the atoms.

A trained CGAN is used in the method of the present invention.

A Generative Adversarial Network (GAN) is a Machine Learning framework that rely on a generator learning a mapping from a noisy representation of the input image (usually a vector) to the output image, and a discriminator that learns to distinguish the images produced by the generator from the target ones. The generator improves the mapping during the training to produce results that cannot be distinguished from the "real" images by the discriminator, which is trained to do its best in detecting generator fakes.. A CGAN is a type of GAN that, in addition to the noisy representation of the input image, uses an additional condition to map the output image. In the present invention, the generator learns a mapping from the observed input image and its vector representation with random noise to the output image.

The method for training the CGAN comprises the following steps:
i) providing a trained CGAN to a data processor device, wherein the trained CGAN comprises:
   - a generator network with an encoder-decoder structure (U-Net architecture) comprising
      o blocks of convolutional layers,
      o pool layers and
      o dropout layers,
   - a discriminator network comprising convolutional and pool layers,
   - and an Image Data Generator;
ii) feeding the generator network with a plurality of constant-height AFM greyscale images of known or predetermined organic molecules, for instance with the AFM images of the database QUAM-AFM, wherein the shape and the contrast of said images and their variation with the height show the 3D positions and the size (chemical nature) of the atoms and the distance between said atoms in the organic molecule, the generator generating a 2D colored RGB structural representation of the positions and the sizes of the atoms and the distance between said atoms in the organic molecule in the form of a ball-and-stick depiction, wherein balls of different colors and sizes represent the different chemical nature of the atoms and sticks represent the bonds between said atoms, and wherein each ball of the representation is centered on the position occupied by the atom it represents in the AFM images,
iii) feeding the discriminator network with the same plurality of constant-height AFM greyscale images of known or predetermined organic molecules used to feed the generator in step (ii) and alternatively with either the ball-and-stick depiction obtained by the generator network in step (ii) or the real ball-and-stick depiction of the known or predetermined molecules, the discriminator superimposing and comparing segmented patches of the images and depictions, and
iv) the discriminator selecting, in a data processor device, the colored 2D colored RGB structural representation in the form of a ball-and-stick depiction from the superimposed segmented patches of the images and depictions, preferably of 16x16 pixels, that match the positions and the sizes of the atoms and the distance between said atoms in step (iii) and disregarding those that does not match the positions and the sizes of the atoms and the distance between said atoms.

Quasar Science Resources S.L. - Universidad Autónoma de Madrid - Atomic Force Microscopy (QUAM-AFM) (https://doi.org/10.21950/UTGMZ7) is used for training the CGAN.

QUAM-AFM is a publicly available dataset of 165 million AFM images theoretically generated from 686,000 isolated molecules using 240 different combinations of AFM operational parameters (10 tip-sample distances, 6 different oscillation amplitudes, and 4 different values for the torsional stiffness of the CO molecule, that are known to depend on the details of the attachment of the molecule to the metal tip apex. QUAM-AFM also provides the ball-and-stick depictions of each molecule generated from the atomic coordinates. These depictions share the same scale used in the AFM images: if we superimpose the two images, each ball of the representation is centered on the position occupied by the atom it represents in the AFM images.

QUAM-AFM dataset includes organic molecules, discarding all other compounds that may not have purely molecular forms, like organic salts or inorganic compounds and polymers. The selected molecules contain the four basic elements of organic chemistry (carbon, hydrogen, nitrogen, and oxygen) plus some other less common elements which are still frequent on organic compounds like sulphur, phosphorus, and the halogen atoms (fluorine, chlorine, bromine, and iodine). The largest molecule in QUAM-AFM database has a total of eighty--five atoms.

Very small molecules, namely, those containing less than eight atoms have been discarded, as due to their extremely high surface mobility and huge variety of adsorption configurations, are not good candidates to be identified solely by means of AFM and therefore are not included in QUAM-AFM database. In addition, very large molecules having a structure that does not fit into a square--based cell with a side length of 24 Å are also not forming part of the QUAM-AFM dataset.

The database QUAM-AFM is restricted to quasi--planar molecules, which display only height variations up to 1.83 Å along the z-axis in order to include aliphatic chains with *sp³* carbon atoms (methyl groups) as side groups. QUAM-AFM comprises a set of molecules that includes aliphatic, cyclic and aromatic compounds, in particular a large number of hydrocarbons (alkanes, alkenes, alkynes, etc.) together with all the traditional organic families (alcohols, thiols, ethers, aldehydes and ketones, carboxylic acids, amines, amides, imines, esters, nitriles, nitro and azo compounds, halocarbons and acyl halides, etc.).

The 686K structures in QUAM-AFM have been split into training, validation and test sets with 581K, 24K and 81K structures respectively. It has to be noticed that, for the testing of CGAN, the test set has been chosen to be particularly large in order to have a sufficient variety of molecular structures and chemical compositions to assess the performance of the CGAN.

During the training of CGAN, the generator, and the discriminator, are confronted against each other in a zero-sum game: the generator learns both to fool the discriminator and to generate images as close as possible to the ball-and-stick depiction, and the discriminator learns to guess whether the second input image is real or fake. This competition forces generator and discriminator to significantly improve their performance during the training. The discriminator is only useful to force the generator to improve. Therefore, once this objective has been achieved, the discriminator is discarded.

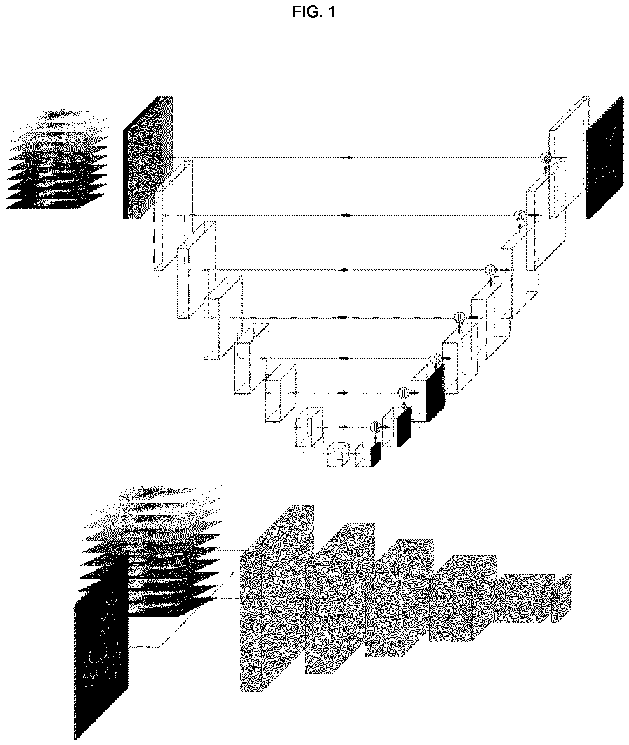

The generator with an encoder-decoder structure is composed of a series of similar blocks where the main difference is the number of kernels applied in each convolution and the dimensions of each input (Figure 1). The input consists of a stack of 10 greyscale AFM images (a single channel), and the corresponding ball-and-stick depiction is the output. The first layers of the generator are a dropout layer with a rate of 0.5 and two 3D convolutional layers to process the image stack. A dropout layer with such a high rate is important for the CGAN to be able to generalize and make accurate predictions when dealing with experimental images. The first 3D convolution includes 64 kernels, each of them has (4,3,3) size and is applied with a stride of (3,1,1) and padding. The second 3D convolution also has 64 kernels but, in this case, the kernels have size (4,4,4) and are applied with a stride of (4,2,2). The output of the second convolutional layer is resized to (128,128,64) and activated with a Leaky ReLU (LReLU) function.

From this point on, the encoder consists of seven blocks (Figure 1). Each block includes a 2D convolution followed by a batch normalisation and a LReLU activation function with α = 0.2. All kernels of the 2D convolution have size (4,4) and are applied with a stride of (2,2). The 2D convolutional layers have 128, 256, 512, 512, 512, 512, and 512 kernels, taking as reference the processing direction from the one closest to the input to the one closest to the compressed representation space. The outputs of the activations are used both to feed the next block of the encoder and to feed the decoder block of the same size. The generator decoder blocks include the following layers: a transposed convolution, a batch normalization, a dropout layer with rate 0.2 (only in the three layers closest to the space of the compressed representation, see Figure 1), a concatenation with the output of the corresponding encoder block, and, finally, a Rectified Linear Unit Activation Function (ReLU) activation (except for the last block, the one closest to the output, that is activated with an hyperbolic tangent function).

The discriminator (Figure 1) consists of a sequence of layers, initiated by a concatenation of all input images (note that we can consider the 10 AFM images as a single image with 10 channels). It is followed by a 2D convolutional layer with 64 kernels of size (4,4) and stride of (2,2) activated with LReLU. Then, it has four blocks consisting of a 2D convolutional layer, a batch normalization and a LReLU activation (α = 0.2). The convolutions have 128, 256, 512 and 512 kernels with size (4,4) and stride (2,2) respectively. The last layer is a 2D convolution with a single kernel of size (4,4) which is activated with the sigmoid function.

During the training of CGAN, the discriminator is feed with the same stack of constant-height AFM greyscale images of known or predetermined organic molecules used to feed the generator and, alternatively, with either the 2D colored RGB structural representation in the form of a ball-and-stick depiction obtained by the generator network in step (a) or the real ball-and-stick depiction of the known or predetermined organic molecules. The discriminator predicts whether the ball-and-stick depiction of the known or predetermined molecule is the ground truth (real) or the ball-and-stick depiction generated by the generator network (fake). This prediction is not achieved by a global assessment of the input data but by comparing them segmented into patches of 16x16 pixels. This local analysis based on small patches of the images makes CGAN especially powerful in AFM image analysis, as the features induced by the structure and composition on the AFM images depend strongly on the local chemical environment and smoothly on the global molecular configuration.

The success or failure in the predictions of both the generator and the discriminator is incorporated into the update of the parameters of generator and discriminator using a standard gradient backward propagation algorithm. The ball-and-stick depiction generated by the generator is compared with the real ball-and-stick depiction and differences are back propagated into the CGAN, improving the performance of both the generator and the discriminator. In the case of the discriminator, its prediction as the generator predicted image (fake) or the true ball-and-stick depiction (real) is compared with the known character of this input in each case, and parameters in the discriminator network are, according to the accuracy of the prediction, reinforced or changed using gradient back propagation. With this training, the generator is forced to learn to generate images as close as possible to the real ball--and--stick depiction in order to fool the discriminator, that is improving its ability to determine the nature (real or fake) of the ball-and--stick depiction shown to it together with the stack of AFM images.

During training of CGAN, one of the 24 combinations of AFM operational parameters (6 different oscillation amplitudes, and 4 different values for the torsional stiffness of the CO molecule) available in QUAM-AFM for each input stack are randomly chosen. This variability in the input data
- makes sure that the parameters with which the AFM experiment has been carried out do not play a decisive role for the CGAN network to succeed in the identification of the structure,
- prevents the overfitting, and
- provides the CGAN network with the ability to generalize.

This variability is further enhanced with the application of an Imaging Data Generator (IDG) to the training set which applies different deformations (zoom, rotations, shifts, flips and shear) to the input images (Figure 2) and normalizes the pixel value. The use of an IDG is motivated by the fact that experimental images have some characteristic features that are not captured by AFM simulations and that could hamper identification. For example, experimental images do not display the full symmetry of the organic molecule. These differences between experimental and theoretical AFM images for a given organic molecule could be due to the unavoidable presence of noise in the experiments, asymmetries in the tip that are not included in the simulation of AFM images, or to the fact that organic molecules relax and deform due to the interaction with the substrate, while we are considering ideal, gas-phase structures in the simulated AFM images used for the training.

The deformations provided by the application of the IDG during the training mimic these effects and contribute significantly to confer the CGAN network with the ability to identify organic molecules from experimental images. The selection of appropriate deformation parameters for the IDG is important as a proper choice considerably increases the accuracy of the identification.

Figure 2 shows a particular example of the application of the IDG and provides information on the range values used for the different operations. In the practical application of the IDG it is important to remember that the ball-and-stick depictions included in QUAM-AFM are proportional (share the same scale) to the AFM images. Thus, the IDG has to be applied to both the input AFM images and the ball-and-stick depiction during the training: i. e., if we rotate the input AFM images, then, the corresponding ball-and-stick depiction must be rotated with the same angle. Otherwise, the atomic positions of the ball-and-stick representation would not match the corresponding atomic positions of the AFM images, and the CGAN would not be able to learn a local translation (from the pixel environment) between the shape and intensity of the AFM image and the type of atom that caused it. This applies to all the operations in the IDG except for the shear, that is not applied to the output ball-and-stick depiction. This is motivated by the fact that shear does not represent a movement of the sample, but a simulation of a possible deformation of the results that could occur in an experiment but should not appear in the prediction.

Regarding the loss functions, the generator of the CGAN was compiled with Mean Absolute Error (MAE), while the binary cross entropy was used for the discriminator. The model was minimised by applying batches of 32 inputs with the Adaptive Moment Estimator (Adam) optimiser, where the learning rate and first moment parameters were set to 2·10⁻⁴ and 0.5 respectively. The training of the CGAN network was carried out during six epochs (109K iterations), displaying 300 predictions of the validation set to estimate the optimal training point every 10.000 iterations.

Given the complexity of the CGAN and the very limited experimental data available, the successful performance of the training of the CGAN training relies on
- the very efficient training provided by the competition of the generator and discriminator,
- the design of the CGAN, that incorporates dropout layers that provide the CGAN with the ability to generalize, and
- the application of an Image Data Generator (IDG) that normalizes the image pixel values and incorporates different random deformations (zoom, rotations, shifts, flips and shear) to mimic features of the experimental images, like the presence of noise and effects associated to the tip asymmetry, that are absent in the AFM simulations and that could hamper identification.

The trained CGAN of the present invention is therefore defined as comprising:
- a generator network with a U-Net based architecture comprising blocks of convolutional layers configured to process a plurality of AFM images and blocks of transpose convolutional layers in order to generate the 2D colored RGB structural representation in the form of a ball-and-stick depiction. The generator further comprising dropout layers included in predetermined blocks of convolutional layers configured to prevent the overfitting during the training with theoretical AFM images and allow the generator to generalize and make accurate predictions when dealing with experimental AFM images
- a discriminator network comprising convolutional layers configured to process together the plurality of AFM images of known or predetermined organic molecules and the colored 2D colored RGB structural representation in the form of a ball-and-stick depiction obtained by the generator network in order to predict whether the ball-and-stick depiction of the known or predetermined organic molecule is the ground truth (real) or the ball-and-stick depiction generated by the generator network (fake)
- and a IDG configured to apply pixel-value normalization, and different random deformations selected from [-15,15] % for zoom, [-180,180] degrees of rotation, vertical and horizontal shift, random vertical and horizontal flips, [-20,20] % for shear and any combination thereof to the theoretical AFM images thereby providing them with features of the experimental images like the presence of noise and effects associated to the tip asymmetry, that are absent in the AFM simulations and that could hamper identification.

Therefore, the first aspect of the invention refers to computer implemented method for identifying an organic molecule from AFM images and for generating a 2D colored RGB structural representation of said organic molecule in the form of a ball-and-stick depiction (herein the method of the invention), said method characterized in that it comprises the following steps:
(a) acquiring a plurality of constant-height AFM greyscale images of the organic molecule at different tip height distances above said organic molecule using a functionalized metal tip apex by means of a Frequency Modulation Atomic Force Microscope (FM-AFM), wherein said different tip height distances range between 280 pm and 370 pm, and wherein the shape and the contrast of said images and their variation with the tip height show the 3D positions, the sizes (chemical nature) of the atoms and the distance between said atoms in the organic molecule;
(b) providing a trained CGAN to a data processor device, wherein the trained CGAN comprises:
   - a generator network with an encoder-decoder structure comprising
      ∘blocks of convolutional layers
      ∘ pool layers and
      ∘dropout layers,
• a discriminator network comprising convolutional and pool layers
• and an Image Data Generator;
and (c) feeding, to the data processor device, the generator network of the trained CGAN network with the AFM greyscale images obtained in step (a), the generator of said trained CGAN network generating a 2D colored RGB structural representation of the positions and the size (chemical nature) of the atoms and the distance between said atoms in the organic molecule in the form of a ball-and-stick depiction, wherein balls of different colors and sizes represent the different chemical species of atoms and sticks represent the bonds between said atoms and wherein each ball of the representation is centered on the position occupied by the atom it represents in the AFM images.

AFM operating in its Frequency Modulation dynamic mode (FM-AFM) allows characterization and manipulation of all kinds of materials at the atomic scale by measuring the change in the frequency of an oscillating tip due to its interaction with the organic molecule sample. When the tip is functionalized with inert closed-shell molecules, in particular a CO molecule, the resolution is enhanced providing access to the inner structure of molecules. The outstanding contrast originates mainly from the Pauli repulsion between the CO probe and the sample molecule. This repulsive force contribution arises because the electron densities of tip and sample overlap, resulting in increasing frequency shifts, which are changes in the oscillation frequency of the cantilever holding the tip due to the tip-sample interaction. Shifts are observed as bright features in the constant height AFM images above atom positions, sizes and bonds (distance between the atoms), reflecting the organic molecular structure.

Therefore, step (a) of the method of the present invention refers to the acquisition of a plurality of constant-height AFM greyscale images of the organic molecule, at different tip height distances above said organic molecule using a functionalized metal tip apex, by means of an FM-AFM, wherein said distances range between 280 pm and 370 pm, and wherein the shape, the contrast of said images and their variation with the tip height is controlled by the electronic charge density of the organic molecule. The charge density of the organic molecule defines the 3D positions and the size (chemical nature) of the atoms and the distance between said atoms in the organic molecule.

The term "a functionalized metal tip apex" refers herein to a tip apex of a metal, usually Cu but other metals like Ag and Pt can also be used, which is functionalized with inert closed shell atoms or molecules that dramatically enhanced the resolution of the AFM images, providing access to the inner structure of the organic molecule. Examples of inert closed shell atoms or molecules are a Xe atom and a CO molecule, respectively.

A CO functionalized metal tip apex is preferred in the present invention since it increases the AFM image contrast because of:
- the Pauli repulsion between the lone pair of the oxygen atom in the CO molecule and the charge density of the sample molecule is very directional, due to the preferential distribution of electronic charge associated with the lone pair along the molecular axis, and
- the CO molecule attached to the metal tip provides a complex electric field, with a very localized central feature, right in front of the O atom and repulsive for the electrons in the sample molecule, that provides a rapidly varying electrostatic interaction, both vertical and laterally, and
- the tilting of the CO molecule significantly enhances the intramolecular features.

Therefore, in a preferred embodiment of the present invention, the functionalized metal tip apex used in step (a) is selected from Cu, Ag or Pt.

In another preferred embodiment of the method of the present invention, the functionalized metal tip apex used in step (a) is functionalized with inert closed shell atoms or molecules. More preferably, the functionalized metal tip apex is functionalized with a Xe atom or a CO molecule.

For acquiring the plurality of constant-height AFM greyscale images different tip height distances above said organic molecule ranging between 280 pm and 370 pm have to be obtained by means of an FM-AFM microscope that allows the acquisition of images with different contrasts as a function of the tip height distances. The shape and the contrast of said images and their variation with the tip height defines the 3D spatial distribution of the electronic charge, that results from the interplay of the chemical species, their chemical environment, and their relative heights with respect to the other atoms in the molecular configuration Therefore, it is essential that a plurality of images is acquired since the whole information regarding said 3D spatial distribution of the electronic charge is therein. Preferably, at least 10 images should be taken in order to properly characterize the FM-AFM contrast in the tip height distance range between 280 pm and 370 pm above said organic molecule. In this tip height range, the Pauli repulsion and the electrostatic interaction between the tip and the organic molecule, vary significantly, leading to strong changes in the contrast of the FM-AFM images that include features that are characteristic of the atoms and their molecular environment. The shape and the contrast of said images and their variation with the tip height contains all the information about the 3D position and the size (chemical nature) of the atoms and the distance between said atoms.

Therefore, in a preferred embodiment of the method of the present invention, a plurality of at least 10 constant-height AFM greyscale images of the organic molecule are acquired in step (a).

In another preferred embodiment of the method of the present invention, step (a) is performed at least 10 different height distances.

Step (b) refers to providing a trained CGAN to a data processor device, wherein the trained CGAN comprises:
- a generator network with an encoder-decoder structure comprising
   o blocks of convolutional layers
   o pool layers and
   o dropout layers included in predetermined blocks,
- a discriminator network comprising convolutional and pool layers,
- and an Image Data Generator.

Step (c) refers to the feed, to the data processor device, the generator network of the trained CGAN with the AFM greyscale images obtained in step (a), the generator of said trained CGAN generating a 2D colored RGB structural representation of the positions and the size (chemical nature) of the atoms and the distance between said atoms in the organic molecule in the form of a ball-and-stick depiction, wherein balls of different colors and sizes represent the different chemical atoms and sticks represent the bonds between said atoms and wherein each ball of the representation is centered on the position occupied by the atom it represents in the AFM images.

Another aspect of the present invention refers to a FM-AFM microscope (herein the microscope of the present invention) comprising a functionalized metal tip apex and configured to carry out step (a) of the method of the invention and a data processing device configured to carry out steps (b) and (c) of the method of the present invention.

In a preferred embodiment of the microscope of the present invention, said microscope further comprises a display unit, connected to the data processing device, and configured to display the colored 2D colored RGB structural representation in the form of a ball-and-stick depiction obtained in step (c) of the method of the present invention.

The microscope of the present invention comprises a functionalized metal tip apex. Preferably the metal of the functionalized metal tip apex is selected from Cu, Ag or Pt.

The functionalized metal tip apex is herein preferably functionalized with inert closed shell atoms or molecules, more preferably the functionalized metal tip apex is functionalized with a Xe atom or a CO molecule. Said functionalized metal tip apexes dramatically enhanced the resolution of the AFM images.

Another aspect of the present invention refers to a computer program (herein the computer program of the invention) comprising instructions which, when the program is executed by a data processing device, cause a data processing device to carry out carry out steps (b) and (c) according to the method of the present invention, in said data processing device.

The last aspect of the invention refers to a computer-readable data carrier having stored thereon the computer program of the present invention.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples and drawings are provided by way of illustration and are not intended to be limiting of the present invention.

### BRIEF DESCRIPTION OF THE DRAWINGS

**FIG. 1****.** Scheme of the trained CGAN of the present invention. The generator has an encoder-decoder structure (U-Net architecture). The encoder includes a first block with a dropout layer (black) and two 3D convolutional layers (gray), and seven blocks of 2D convolutional and pool layers. The decoder includes eight blocks with 2D transposed convolutional layers, with a final dropout layer (black) in the first three blocks. The discriminator includes a first block with a concatenation layer of all input images followed by a 2D convolutional layer, and four blocks consisting of a 2D convolutional layer, a pool layer, a batch normalization and a LReLU activation. The last layer is a 2D convolution which is activated with the sigmoid function.
**FIG. 2****.** Image Data Generator (IDG) applied to a AFM input image and the ball- and-stick depiction of the molecule 6,14,22-triethynyltetracyclo[18.4.0.04,9.012,17] tetracosa-1(20),4(9),5,7,12(17),13,15,21,23-nonaen-2,10,18-triyne. The parameters selected for the IDG during the training are randomly chosen in the range of [-180,180] degrees for rotation, [- 15,15] % for zoom and for vertical and horizontal shift, [-20,20]% for shear and random vertical and horizontal flip.

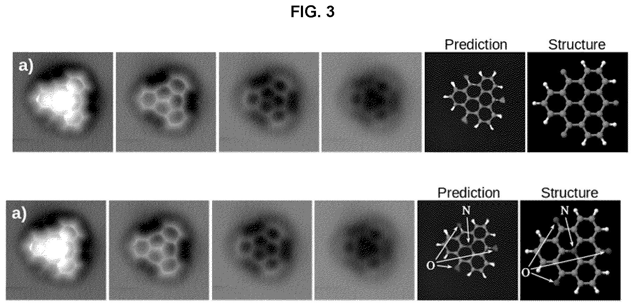

**FIG. 3****.** (From left to right) the four first images correspond to the experimental AFM images for
   1-azahexacyclo[11. 7.1.1^{3,19}.0^{2,7}.0^{9,21}.0^{15,20}]docosa-2,4,6,9(21),10,12,15,17,19-nonaene-8,14,22-trione. The 5^{th} image refers to the 2D colored RGB structural representation of said molecule obtained following the method of the invention, The color code for the balls representing the chemical species is: carbon (grey), hydrogen (white), oxygen (red), and nitrogen (blue). The 6^{th} image refers to a optimized ball-and-stick depiction of the molecule. The bottom panel provides additional grey scale versions of the 5th and 6th images where the position and chemical nature of the atoms different from carbon and hydrogen (oxygen (O) and nitrogen (N)) are indicated by arrows.

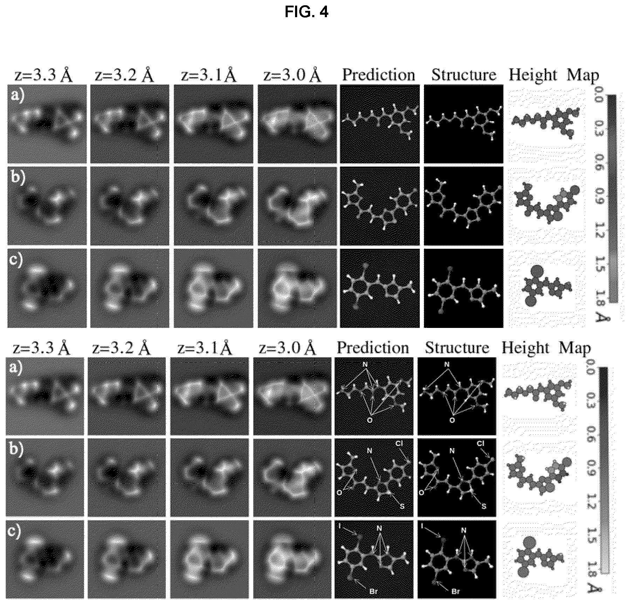

**FIG. 4****.** (From left to right) four AFM images at different tip-sample distances, 2D colored RGB structural representation obtained following the method of the invention, real structure, and height map for (a) 2-(2-aminoethoxy)-N-(3,5-dimethoxyphenyl)acetamide, (b) 3-[2-(4- chlorophenyl)-1,3-thiazol-4-yl]-1-(5-methylfuran-2-yl)prop-2-en-1-one and (c) N-(5-bromo-2- iodophenyl)-5-methyl-1H-imidazol-2-amine. The bottom panel provides additional grey scale versions of the 5th and 6th images where the position and chemical nature of the atoms different from carbon and hydrogen (oxygen (O), nitrogen (N), sulfur (S), chlorine (CI), bromine (Br) and iodine (I)) are indicated by arrows.

### EXAMPLES

### EXAMPLE 1

We have selected a set of AFM images already available in the literature (N. J. van der Heijden, et al., ACS Nano 10, 8517 (2016)). This structure is not part of the training set, so, this was a perfect example to verify the ability of the method of the present invention to generalize to other molecules not included in the training set. Only 4 images corresponding to different tip-sample distances were published in this paper, so we have linearly interpolated the images two by two to extract additional images to complete the input, a stack of at least 10 images. It is important to stress that the interpolated images are generated for the sole purpose of completing the input dimensions, i.e. they do not provide additional information to that supplied by the original images.

Figure 3 shows experimental AFM images, taken at constant height and acquired with a CO-terminated tip, for a 1-azahexacyclo[11.7.1.13,19.02,7.09,21.015,20]docosa-2,4,6,9(21),10,12,15,17,19-nonaene-8,14,22-trione molecule adsorbed on a Cu(111) surface. These experimental AFM images show an imperfect threefold symmetry that, according to the literature (N. J. van der Heijden, et al., ACS Nano 10, 8517 (2016)), is caused by the flexibility of the CO--Cu bond coupled with an asymmetric tip. Therefore, the test with these experimental images is really tough: We are not only severely reducing the amount of information provided to the CGAN network but we are feeding it with images containing characteristic features induced by asymmetrical tips that have not been considered in the theoretical AFM simulations used for the training.

The method of the present invention is not only able to reveal the molecular structure but also to predict with perfect accuracy the chemical species that make up the molecule.

### EXAMPLE 2: Generation of a ball-and-stick depiction from theoretically simulated AFM images taken from the QUAM-AFM data set

This second example demonstrates the ability of our method to identify both chemically and structurally non-flat molecules that include most of the relevant chemical species in organic chemistry. In this case, we feed the model with theoretically simulated AFM images taken from the QUAM-AFM data set. None of the three molecules considered here have been used to train the CGAN. For each molecule, we consider a stack of 10 constant-height AFM images calculated with a random selection of the simulation parameters among the 24 possible combinations offered by QUAM-AFM.

Figure 4 shows some of the AFM images included in the stack for each of the molecules. The images are extraordinarily complex due to the interplay of the chemical nature of the atoms and the non-planar character of the molecules, with atoms at different heights due to internal rotations induced by steric hindrance effects and to the presence of methyl groups. Given this complexity, a human expert would not be able to identify these molecules from the observation of the AFM images.

Figure 4 (a) shows the identification of 2-(2-aminoethoxy)-N-(3,5-dimethoxyphenyl) acetamide, a really tough example. The corresponding AFM images are characterised by strong distortions of the structure created by the strong charge accumulation around the oxygen atoms. These strongly electronegative atoms hide their bonds with the sp3 carbons, creating a triangular feature at the position of the hexagonal ring, and hiding also the presence of the N atom attach to it. Nevertheless, the model is able to differentiate the presence of *sp3* and sp2 carbons and identify the two amino groups, leading to a perfect prediction.

Figure 4 (b) corresponds to another difficult case, 3-[2-(4-chlorophenyl)-1,3-thiazol-4-yl]-1-(5-methylfuran-2-yl)prop-2-en-1-one. Again, looking at each individual AFM images, it is impossible to recognize the combination of hexagonal and pentagonal rings that constitute the non-planar structure of the molecule, let alone the composition, where we have carbon substitutions by sulphur, oxygen and nitrogen atoms, and the presence of a chlorine, replacing one of the H atoms, and a methyl group. However, thanks to the change with the tip height of the contrast in the AFM images of the features associated with these atoms in their different local chemical environments, that our CGAN has learned during the training, the ball-and-stick pre- diction is exact, completely indistinguishable of the real one, providing a complete structural and compositional identification of the molecule.

The case presented in Figure 4 (c), N-(5-bromo-2-iodophenyl)-5-methyl-1H-imidazol-2-amine, adds another extra difficulty, with the challenge to discriminate between two different halogen atoms, bromine and iodine. Figure 4 (b) and (c) shows that halogen atoms attached to a benzene ring, replacing a H atom, appear in the AFM images as a very characteristic feature: an elongated bright oval, perpendicular to the halogencarbon bond. We have shown (J. Tschakert et al., Nat. Comm. 11, 5630 (2020)) that this particular shape reflects the strongly anisotropic charge distribution of a halogen (X) that is covalently bound to an organic molecule (R). Typically, a "belt" of high electron density is observed around the X-R bond axis, while the electron density at the cap of the halogen, the so-called σ-hole, is significantly lower and can be even positive. Although the quantitative details of the distribution are different depending on the type of halogen and the organic rest, the shape of the charge density distribution is common to all the halogens. The electrostatic potential associated with this charge distribution reveals negative oval shaped areas at the halogen atoms. These repulsive regions are in concordance with the "negative belts" around the halogens. The surface potential at the caps of the halogens (at the σ-holes) is less negative (less repulsive). The relaxation of the probe enhances the asymmetry introduced by the electrostatic potential resulting in oval shaped features in the HR- AFM images at the halogens.

These characteristic oval contrast features, that are a direct fingerprint of the σ-holes of the halogens, are very conspicuous one the AFM images and could be easily spotted by a human expert. However, it would be extremely challenging for this expert to identify which particular halogen is present. Our CGAN has learned during the training the details of the contrast and its evolution with the tip height associated with each halogen atom (illustrated in the AFM images in Figure 2 (b) and (c), leading to a perfect identification of the halogens appearing in these two molecules (Cl in fig. 4 (b) and I and Br in fig. 4 (c)).

## Claims

1. A computer implemented method for identifying an organic molecule from Atomic Force Microscopy images and for generating a 2D colored RGB structural representation of said molecule in the form of a ball-and-stick depiction, said method **characterized in that** it comprises the following steps:
(a) acquiring a plurality of constant-height Atomic Force Microscopy greyscale images of the organic molecule at different height distances above said organic molecule using a functionalized metal tip apex by means of a Frequency Mode Atomic Force Microscope microscope, wherein said different height distances range between 280 pm and 370 pm, and wherein the shape, the contrast of said images and their variation with the tip height show the 3D positions, the sizes of the atoms and the distance between said atoms in the organic molecule;
(b) providing a trained CGAN to a data processor device, wherein the trained CGAN comprises:
• a generator network with an encoder-decoder structure comprising
∘ blocks of convolutional layers
∘ pool layers and
∘ dropout layers ,
• a discriminator network comprising convolutional and pool layers
• and an Image Data Generator;
and (c) feeding, to the data processor device, the generator network of the trained CGAN with the Atomic Force Microscopy greyscale images obtained in step (a), the generator of said trained CGAN network generating a 2D colored RGB structural representation of the positions and the sizes of the atoms and the distance between said atoms in the organic molecule in the form of a ball-and-stick depiction, wherein balls of different colors and sizes represent the different chemical atoms and sticks represent the bonds between said atoms and wherein each ball of the representation is centered on the position occupied by the atom it represents in the AFM images.

2. The method according to claim 1, wherein a plurality of at least 10 constant-height Atomic Force Microscopy greyscale images of the organic molecule are acquired in step (a).

3. The method according to any of claims 1 or 2, wherein step (a) is performed at, at least, 10 different height distances.

4. The method according to any of claims 1 to 3, wherein the functionalized metal tip apex used in step (a) is selected from Cu, Ag or Pt.

5. The method according to any of claims 1 to 4, wherein the functionalized metal tip apex used in step (a) is functionalized with inert closed shell atoms or molecules.

6. The method according to claim 5, wherein the functionalized metal tip apex used in step (a) is functionalized with a Xe atom or a CO molecule.

7. A Frequency Modulation Atomic Force Microscopy microscope comprising a functionalized metal tip apex and configured to carry out step (a) of the method according to claims 1 to 6 and a data processing device configured to carry out steps (b) and (c) of the method according to claims 1 to 6.

8. The FM-AFM microscope according to claim 7, further comprising a display unit, connected to the data processing device, and configured to display the 2D colored RGB structural representation in the form of a ball-and-stick depiction obtained in step (c) of the method according to claims 1 to 6.

9. The FM-AFM microscope according to any of claims 7 and 8, wherein the metal of the functionalized metal tip apex is selected from Cu, Ag or Pt.

10. The FM-AFM microscope according to any of claims 7 to 9, wherein the functionalized metal tip apex is functionalized with inert closed shell atoms or molecules, preferably the functionalized metal tip apex is functionalized with a Xe atom or a CO molecule.

11. A computer program comprising instructions which, when the program is executed by a data processing device, cause a data processing device to carry out steps (b) and (c) according to the method of claims 1 to 6, in said data processing device.

12. A computer-readable data carrier having stored thereon the computer program of claim 11.
